# EUROPEAN PATENT APPLICATION

(11) **EP 4 212 226 A1**
(43) Date of publication of application: **19.07.2023**
(21) Application number: 22151845.9
(22) Date of filing: 17.01.2022
(51) Int. Cl.: B01D 3/14, C07C 51/44, C07C 53/126, C07C 57/12, C11C 1/00, C11C 1/10

(54) **PLANT AND PROCESS FOR FRACTIONATING FATTY ACID MIXTURES**

(71) Applicant: Sulzer Management AG, 8401 Winterthur (CH)
(72) Inventor: KEK, Wan Seang, Shanghai (CN); LUI, Keen Hoe, Singapore (SG); YANG, Quan, Singapore (SG); FARQUET, Patrick, 8185 Winkel (CH)
(74) Representative: Henkel & Partner mbB

(57) **Abstract**

A method for fractionating a crude composition comprising at least six fatty acids having each a different chain length into at least four fractions, comprises the following steps:
i) feeding the crude composition into a first distillation column and distilling it so as to obtain at least an overhead fraction and a bottom fraction,
ii) feeding the bottom fraction of the first distillation column to a second distillation column and distilling it so as to obtain at least an overhead fraction, at least two side fractions and a bottom fraction,
iii) feeding the bottom fraction of the second distillation column to a third distillation column and distilling it so as to obtain at least an overhead fraction, at least two side fractions and a bottom fraction and
iv) feeding the bottom fraction of the third distillation column to a fourth distillation column and distilling it so as to obtain at least an overhead fraction, a side fraction and a bottom fraction,
wherein at least one of the first distillation column, the second distillation column and the third distillation column is a middle dividing-wall column, from which also at least one side fraction is obtained.

## Description

The present invention relates to a plant and process for fractionating fatty acid mixtures, such as in particular for fractionating palm kernel fatty acid or coconut fatty acid.

A plurality of natural occurring fatty acid mixtures, such as palm kernel fatty acid and coconut fatty acid, are complex mixtures comprising several fatty acids having different chain lengths. For instance, palm kernel fatty acid and coconut fatty acid comprise several even-chained fatty acids, i.e. monocarboxylic acids, namely C₆-, C₈-, C₁₀-, C₁₂-, C₁₄-, C₁₆-, C₁₈- and longer fatty acids. More specifically, palm kernel fatty acid comprises 0.3% by weight of C₆-fatty acids, 4.4% by weight of C₈-fatty acids, 3.7% by weight of C₁₀-fatty acids, 48.2% by weight of C₁₂-fatty acids, 15.6% by weight of C₁₄-fatty acids, 7.8% by weight of C₁₆-fatty acids, 19.8% by weight of C₁₈-fatty acids and 0.2 of other compounds, such as non-fatty acid impurities, for instance ketones, alkenes or the like. The C₁₈-fatty acid fraction comprises the saturated C₁₈-stearic acid, the mono-unsaturated C₁₈-oleic acid and the di-unsaturated C₁₈-linoleic acid. In order to obtain the single fatty acids from these mixtures, the respective fatty acid mixtures must be fractionated.

This fractionation is usually performed by distillation, wherein 7 distillation columns are needed to obtain 7 fractions from palm kernel fatty acid, namely the C₆-, C₈-, C₁₀-, C₁₂-, C₁₄-, C₁₆- and C₁₈ₚₗᵤₛ-fatty acid fractions. More specifically, the palm kernel fatty acid mixture is distilled in a first distillation column so as to obtain as overhead fraction a rather impure C₆-fatty acid fraction, as side fraction a C₈₋₁₀-fatty acid fraction and a bottom fraction with the remaining fatty acids. The C₆- and C₈₋₁₀-fatty acid fractions are further purified in two (i.e. the second and third) distillation columns which are erected under campaign running mode, to a rather pure C₆-fatty acid fraction, a rather pure C₈-fatty acid fraction and a rather pure C₁₀-fatty acid fraction. The bottom fraction of the first distillation column is distilled in a fourth distillation column so as to obtain as side fraction a C₁₂-fatty acid fraction and a bottom fraction with the remaining fatty acids, wherein the bottom fraction of the fourth distillation column is distilled in a fifth distillation column so as to obtain as side fraction a C₁₄-fatty acid fraction and a bottom fraction with the remaining fatty acids, wherein the bottom fraction of the fifth distillation column is distilled in a sixth distillation column so as to obtain as side fraction a C₁₆-fatty acid fraction and a bottom fraction with the remaining fatty acids, and wherein the bottom fraction of the sixth distillation column is distilled in a seventh distillation column so as to obtain as side fraction a C₁₈ₚₗᵤₛ-fatty acid fraction. Due to the seven distillation columns, the prior art method has a comparably high energy consumption. Moreover, the method is not continuous on account of the campaign mode. In addition, a plant with seven distillation columns requires a large plot area.

It has been already proposed to perform such a fractionation in six distillation columns connected in series with each other. In the first distillation column, a C₆-fatty acid side fraction and another C₈-fatty acid side fraction are obtained, whereas the bottom product is sent to a second distillation column in which a C₁₀-fatty acid side fraction is obtained. The bottom product of the second distillation column is sent to a third distillation column in which a C₁₂-fatty acid side fraction is obtained, whereas the bottom fraction of the third distillation column is sent to a fourth distillation column in which a C₁₄-fatty acid side fraction is obtained, the bottom fraction of the fourth distillation column is sent to a fifth distillation column in which a C₁₆-fatty acid side fraction is obtained and the bottom fraction of the fifth distillation column is sent to a sixth distillation column in which a C₁₈ₚₗᵤₛ-fatty acid side fraction is obtained. However, also this method has a comparably high energy consumption and requires a comparably large plot area. Moreover, the purities of the single fractions is less than those of the fractions obtained with the aforementioned seven columns-method.

In view of all this, the object underlying the present invention is to provide a method and a plant for fractionating fatty acid mixtures, such as in particular palm kernel fatty acid or coconut fatty acid, which is continuous, which leads to fractions with a high purity, which only requires a comparably small plot area and which has, relative to the obtained purity, a comparably low energy consumption.

In accordance with the present invention, this object is satisfied by providing a method for fractionating a crude composition comprising at least six fatty acids having each a different chain length into at least four fractions, wherein the method comprises the following steps:
i) feeding the crude composition into a first distillation column and distilling it so as to obtain at least an overhead fraction and a bottom fraction,
ii) feeding the bottom fraction of the first distillation column to a second distillation column and distilling it so as to obtain at least an overhead fraction and a bottom fraction,
iii) feeding the bottom fraction of the second distillation column to a third distillation column and distilling it so as to obtain at least an overhead fraction and a bottom fraction and
iv) feeding the bottom fraction of the third distillation column to a fourth distillation column and distilling it so as to obtain an overhead fraction, at least one side fraction and a bottom fraction,
wherein at least one of the first distillation column, the second distillation column and the third distillation column is a middle dividing-wall column, from which also at least one side fraction is obtained.

This solution bases on the finding that by replacing one or two or even three distillation columns of the prior art methods by one or two or even three middle dividing-wall distillation columns, not only the energy consumption can be significantly decreased in a continuous process, namely - depending on the specific embodiment by 10 to 25% -, but that also the purity of the obtained fatty acid fractions is improved. More specifically, the method in accordance with the present invention allows to produce C₆-, C₈-,C₁₀-, C₁₂-, C₁₄- and C₁₆-fractions having a purity of at least 99.5% each and a C₁₈ₚₗᵤₛ-fraction containing unsaturated, monounsaturated and di-unsaturated C₁₈-fatty acids (i.e. C_{18:0}-, C_{18:1}- and C_{18:2}-fatty acids) and fatty acids having a longer chain length having a purity of at least 99.0%, and this, as set out above, with a reduced energy consumption. In addition, because of the reduced number of distillation columns, the required plot area for the plant is reduced. Moreover, the total residence time is excellently short and the yield is excellently high.

The numeration of the distillation columns is independent from the nature of the distillation columns. Thus, if there is a series of a non-dividing wall distillation column, a middle dividing-wall distillation column, a further non-dividing wall distillation column and a further middle dividing-wall distillation column these are numer-ated as first non-dividing wall distillation column, as second middle dividing-wall distillation column, as third non-dividing wall distillation column and as fourth middle dividing-wall distillation column.

According to a first particular preferred embodiment of the present invention, the method comprises the use of one middle-wall dividing-wall column. The middle-wall dividing-wall column is preferably any of the first distillation column, of the second distillation column and of the third distillation column. It is more preferred in this embodiment that the middle-wall dividing-wall column is the first or second distillation column.

According to a first variant of this first particular preferred embodiment of the present invention, the method comprises the following steps:
i) feeding the crude composition into a first non-dividing-wall distillation column and distilling it so as to obtain at least an overhead fraction, a side fraction and a bottom fraction,
ii) feeding the bottom fraction of the first non-dividing-wall distillation column to a second middle dividing-wall distillation column and distilling it so as to obtain at least an overhead fraction, at least two side fractions and a bottom fraction,
iii) feeding the bottom fraction of the second middle dividing-wall distillation column to a third non-dividing-wall distillation column and distilling it so as to obtain at least an overhead fraction, a side fraction and a bottom fraction and
iv) feeding the bottom fraction of the third non-dividing-wall distillation column to a fourth non-dividing-wall distillation column and distilling it so as to obtain at least an overhead fraction, a side fraction and a bottom fraction.

According to a second variant of this first particular preferred embodiment of the present invention, the method comprises the following steps:
i) feeding the crude composition into a first middle dividing-wall distillation column and distilling it so as to obtain at least an overhead fraction, at least two side fractions and a bottom fraction,
ii) feeding the bottom fraction of the first middle dividing-wall distillation column to a second non-dividing-wall distillation column and distilling it so as to obtain at least an overhead fraction, a side fraction and a bottom fraction,
iii) feeding the bottom fraction of the second non-dividing-wall distillation column to a third non-dividing-wall distillation column and distilling it so as to obtain at least an overhead fraction, a side fraction and a bottom fraction and
iv) feeding the bottom fraction of the third non-dividing-wall distillation column to a fourth non-dividing-wall distillation column and distilling it so as to obtain at least an overhead fraction, a side fraction and a bottom fraction.

The method of this first particular preferred embodiment of the present invention particularly allows to fractionate a fatty acid mixture comprising at least six fatty acids having each a different chain length in particular into five fractions of which four fractions are very pure, such as to fractionate palm kernel fatty acid or coconut fatty acid into a very pure C₁₂-fatty acid fraction having a purity of at least 99.5% by weight, a very pure C₁₄-fatty acid fraction having a purity of at least 99.5% by weight, a very pure C₁₈-fatty acid fraction having a purity of at least 99.5% by weight, a very pure C₁₈ₚₗᵤₛ-fatty acid fraction having a purity of at least 99.0% by weight and a C₈₋₁₀-fatty acid fraction having a purity of at least 95.0% by weight.

According to a second particular preferred embodiment of the present invention, the method comprises the use of two middle-wall dividing-wall columns. Preferably, the two middle-wall dividing-wall columns are the second and third distillation columns.

According to a variant of this second particular preferred embodiment of the present invention, the method comprises the following steps:
i) feeding the crude composition into a first non-dividing-wall distillation column and distilling it so as to obtain at least an overhead fraction, a side fraction and a bottom fraction,
ii) feeding the bottom fraction of the first non-dividing-wall distillation column to a second middle dividing-wall distillation column and distilling it so as to obtain at least an overhead fraction, at least two side fractions and a bottom fraction,
iii) feeding the bottom fraction of the second middle dividing-wall distillation column to a third middle dividing-wall distillation column and distilling it so as to obtain at least an overhead fraction, at least two side fractions and a bottom fraction and
iv) feeding the bottom fraction of the third middle dividing-wall distillation column to a fourth non-dividing-wall distillation column and distilling it so as to obtain at least an overhead fraction, a side fraction and a bottom fraction.

The method of this second particular preferred embodiment of the present invention particularly allows to fractionate a fatty acid mixture comprising at least six fatty acids having each a different chain length in particular into seven fractions of which six fractions are very pure, such as to fractionate palm kernel fatty acid or coconut fatty acid into a very pure C₈-fatty acid fraction having a purity of at least 99.5% by weight, a very pure C₁₀-fatty acid fraction having a purity of at least 99.5% by weight, a very pure C₁₂-fatty acid fraction having a purity of at least 99.5% by weight, a very pure C₁₄-fatty acid fraction having a purity of at least 99.5% by weight, a very pure C₁₆-fatty acid fraction having a purity of at least 99.5% by weight, a very pure C₁₈ₚₗᵤₛ-fatty acid fraction having a purity of at least 99.0% by weight and a C₆-fatty acid fraction having a purity of at least 95.0% by weight.

In order to allow to increase the purity of the seventh fraction and thus to allow to fractionate a fatty acid mixture into seven different fractions with each having an excellently high purity, it is suggested according to a third particular preferred embodiment of the present invention that the method comprises the use of three or even more middle-wall dividing-wall columns. Preferably, the method of this third particular preferred embodiment of the present invention comprises in addition to the steps of the second particular preferred embodiment of the present invention the step v) of feeding the overhead fraction of the first distillation column to a fifth middle dividing-wall distillation column and distilling it so as to obtain an overhead fraction, at least two side fractions and a bottom fraction.

Thus, it is preferred in the third particular preferred embodiment of the present invention that the method comprises the following steps:
i) feeding the crude composition into a first non-dividing-wall distillation column and distilling it so as to obtain at least an overhead fraction and a bottom fraction,
ii) feeding the bottom fraction of the first non-dividing-wall distillation column to a second middle dividing-wall distillation column and distilling it so as to obtain at least an overhead fraction, at least two side fractions and a bottom fraction,
iii) feeding the bottom fraction of the second middle dividing-wall distillation column to a third middle dividing-wall distillation column and distilling it so as to obtain at least an overhead fraction, at least two side fractions and a bottom fraction,
iv) feeding the bottom fraction of the third middle dividing-wall distillation column to a fourth non-dividing-wall distillation column and distilling it so as to obtain at least an overhead fraction, a side fraction and a bottom fraction, and
v) feeding the overhead fraction of the first distillation column to a fifth middle dividing-wall distillation column and distilling it so as to obtain an overhead fraction, at least two side fractions and a bottom fraction.

The third particular preferred embodiment of the present invention allows to fractionate a fatty acid mixture in particular into seven fractions, with all seven fractions being very pure, such as to fractionate palm kernel fatty acid or coconut fatty acid into a very pure C₆-fatty acid fraction having a purity of at least 99.5% by weight, a very pure C₈-fatty acid fraction having a purity of at least 99.5% by weight, a very pure C₁₀-fatty acid fraction having a purity of at least 99.5% by weight, a very pure C₁₂-fatty acid fraction having a purity of at least 99.5% by weight, a very pure C₁₄-fatty acid fraction having a purity of at least 99.5% by weight, a very pure C₁₆-fatty acid fraction having a purity of at least 99.5% by weight and a very pure C₁₈ₚₗᵤₛ-fatty acid fraction having a purity of at least 99.0% by weight.

In accordance with the present invention, the method uses at least one, preferably at least two and more preferably at least three middle dividing-wall distillation columns. A middle dividing-wall distillation column is a distillation column, which comprises a wall partially separating or dividing, respectively, the distillation column into two parts, in particular into two halves. More specifically, the dividing-wall extends at least essentially vertically and is located - seen in the vertical direction - in the middle of the distillation column so as to divide the middle portion of the distillation column into two parts, however leaving the uppermost and lowermost part of the distillation column undivided. Preferably, at least one and preferably each of the middle dividing-wall distillation columns comprise a dividing wall extending, seen from the bottom to the top of the middle dividing-wall distillation column, from a point being located at 10 to 40% of the distance from the bottom to the top of the middle dividing-wall distillation column to a point being located at 50 to 90% of the distance from the bottom to the top of the middle dividing-wall distillation column. The dividing wall is oriented at least essentially vertically downwards so that the middle dividing-wall distillation column comprises above the dividing wall a top section functioning as refinement distillation section, below the dividing wall a bottom section functioning as distillation section, on one side of the dividing wall a first middle section functioning as feed section and on the opposite side of the dividing wall a second middle section functioning as lateral extraction section. Essentially vertically downwards means in this connection that the angle between the dividing wall and the length axis of the first dividing-wall distillation column (which corresponds to the vertical direction) is at most 20°, preferably at most 10°, more preferably at most 5° and most preferably 0°.

More preferably, the dividing wall of at least one of the middle dividing-wall distillation columns and preferably of all of the middle dividing-wall distillation columns extends, seen from the bottom to the top of the middle dividing-wall distillation column, from a point being located at 20 to 40% of the distance from the bottom to the top of the middle dividing-wall distillation column to a point being located at 50 to 80% of the distance from the bottom to the top of the middle dividing-wall distillation column. Most preferably, the dividing wall of at least one of the middle dividing-wall distillation columns and preferably of all of the middle dividing-wall distillation columns extends, seen from the bottom to the top of the middle dividing-wall distillation column, from a point being located at 25 to 35% of the distance from the bottom to the top of the middle dividing-wall distillation column to a point being located at 55 to 75% of the distance from the bottom to the top of the middle dividing-wall distillation column.

The length of dividing wall relative to the height of the middle dividing-wall distillation column is for at least one middle dividing-wall distillation column and preferably for all middle dividing-wall distillation columns 20 to 80%, i.e. in at least one and preferably each of the middle dividing-wall distillation columns the dividing wall extends over 20 to 80% of the height of the middle dividing-wall distillation column. The height of the middle dividing-wall distillation column is defined in the present invention as the straight distance between the top and the bottom of the middle dividing-wall distillation column. More preferably, in at least one and preferably each of the middle dividing-wall distillation columns the dividing wall extends over 30 to 70% of the height of the middle dividing-wall distillation column and most preferably, in at least one and preferably each of the middle dividing-wall distillation columns the dividing wall extends over 30 to 60% of the height of the middle dividing-wall distillation column.

Good results are in particular obtained, when the method in accordance with the present invention is performed so that at least one and preferably each of the middle dividing-wall distillation columns is operated so that it has 25 to 60 theoretical stages.

In accordance with a further preferred embodiment of the present invention, the method in accordance with the present invention is performed so that at least one and preferably each of the middle dividing-wall distillation columns is operated so that its top section comprises during the operation 5 to 15 theoretical stages, its middle section comprises 10 to 30 theoretical stages and its bottom section comprises 10 to 15 theoretical stages.

Preferably, the feed of at least one and preferably each of the middle dividing-wall distillation columns is fed into the first middle section of the respective middle dividing-wall distillation column.

It is further preferred that from at least one and preferably each of the middle dividing-wall distillation columns one of the at least two side fractions is withdrawn from the top section and another one of the at least two side fractions is withdrawn from the second middle section of the respective middle dividing-wall distillation column.

In a further development of the idea of the present invention, it is proposed to withdraw during the method from at least one and preferably each of the middle dividing-wall distillation columns a purge in the top section of the middle dividing-wall distillation column between the side fraction being withdrawn from the top section and the side fraction being withdrawn from the second middle section of the middle dividing-wall distillation column.

Split-fatty acid crude material always contains in addition to the main fatty acid components certain amounts of impurity components, such as ketones, alkanes, odd-chained fatty acids. In order to get rid of these impurities, it is preferred that appropriate proportions of purge are drawn from the middle dividing-wall distillations as light end from the top and as middle purge between the main fatty acid product.

Good results are in particular obtained, when during the method in at least one and preferably each of the middle dividing-wall distillation columns the reflux ratio is adjusted to be between 1 and 70. For this purpose, as common, at least one and preferably each of the middle dividing-wall distillation columns has an overhead condenser being in fluid communication with the head and a reboiler being in fluid communication with the bottom of the middle dividing-wall distillation column. Likewise, preferably at least one and preferably each of the non-dividing-wall distillation columns has an overhead condenser being in fluid communication with the head and a reboiler being in fluid communication with the bottom of the non-dividing-wall distillation column.

For instance, the reflux ratio in the second middle dividing-wall column of the first variant of the first particular preferred embodiment of the present invention is preferably adjusted to be 1.5 to 3.0, such as about 2.3. Moreover, it is preferred that the reflux ratio in the second middle dividing-wall column of the second particular preferred embodiment of the present invention is adjusted to be 20 to 40, such as about 31.5, and that the reflux ratio in the third middle dividing-wall column of the second particular preferred embodiment of the present invention is adjusted to be 3.5 to 7.0, such as about 5.1. In addition, it is preferred that the reflux ratio in the second middle dividing-wall column of the third particular preferred embodiment of the present invention is adjusted to be 20 to 40, such as about 31.5, that the reflux ratio in the third middle dividing-wall column of the third particular preferred embodiment of the present invention is adjusted to be 3.5 to 7.0, such as about 5.1, and that the reflux ratio in the fifth middle dividing-wall column of the third particular preferred embodiment of the present invention is adjusted to be 35 to 70, such as about 45.

In addition, it is preferred that during the method in at least one and preferably each of the middle dividing-wall distillation columns the pressure is adjusted to be between 0.5 and 10 KPa.

For instance, the pressure in the second middle dividing-wall column of the first variant of the first particular preferred embodiment of the present is preferably adjusted to be 1.0 to 2.0 KPa, such as about 1.5 KPa. Moreover, it is preferred that the pressure in the second middle dividing-wall column of the second particular preferred embodiment of the present invention is adjusted to be 2.5 to 5.0 KPa, such as about 4.0 KPa, and that the pressure in the third middle dividing-wall column of the second particular preferred embodiment of the present invention is adjusted to be 0.5 to 1.5 KPa, such as about 0.8 KPa. In addition, it is preferred that the pressure in the second middle dividing-wall column of the third particular preferred embodiment of the present invention is adjusted to be 2.5 to 5.0 KPa, such as about 4.0 KPa, that the pressure in the third middle dividing-wall column of the third particular preferred embodiment of the present invention is adjusted to be 0.5 to 1.5 KPa, such as about 0.8 KPa, and that the pressure in the fifth middle dividing-wall column of the third particular preferred embodiment of the present invention is adjusted to be 5.0 to 10.0 KPa, such as about 8.0 KPa.

In principle, the method in accordance with the present invention is suitable to be used for fractionating any crude composition comprising at least six fatty acids having each a different chain length into at least four fractions. More preferably, the method in accordance with the present invention is for fractionating any crude composition comprising at least six fatty acids having each a different chain length into at least five fractions and even more preferably into at least six fractions. Most preferably, the method in accordance with the present invention is for fractionating any crude composition comprising at least seven fatty acids having each a different chain length into at least four fractions, more preferably at least five fractions, still more preferably at least six fractions and most preferably at least seven fractions.

Particularly suitable is the method in accordance with the present invention for fractionating palm kernel fatty acid and/or coconut fatty acid into five, six or preferably seven fractions. Therefore, it is preferred that the crude composition contains palm kernel fatty acid and/or coconut fatty acid and more preferred that the crude composition consists of palm kernel fatty acid and/or coconut fatty acid.

The method in accordance with the present invention allows in the aforementioned embodiments to fractionate the palm kernel fatty acid and/or coconut fatty acid into four, five, six or seven fractions having an excellently high purity.

More specifically, it allows to withdraw from the second middle dividing-wall distillation column of the first particular preferred embodiment, which comprises one middle dividing-wall distillation column, wherein this middle dividing-wall distillation column is the second distillation column, as side fraction - preferably from the top section - a C₁₂-fraction having a content of C₁₂-fatty acids of at least 98.0% by weight, whereas a C₁₄-fraction having a content of C₁₄-fatty acids of at least 98.0% by weight is withdrawn as side fraction - preferably from the second middle section - of the second middle dividing-wall distillation column, a C₁₆-fraction having a content of C₁₆-fatty acids of at least 98.0% by weight is withdrawn as side fraction of the third distillation column and a C₁₈ₚₗᵤₛ-fraction having a content of C₁₈- and longer fatty acids of at least 95.0% by weight is withdrawn as side fraction from the fourth distillation column. In addition, as side fraction from the first distillation column a combined C₈₋₁₀-fraction having a content of C₈₋₁₀-fatty acids of at least 98.0% by weight is withdrawn. More preferably, during this embodiment a C₁₂-fraction having a content of C₁₂-fatty acids of at least 99.0% by weight, more preferably of at least 99.5% by weight and most preferably of at least 99.9% by weight is withdrawn as side fraction from the second middle dividing-wall distillation column, whereas a C₁₄-fraction having a content of C₁₄-fatty acids of at least 99.0% by weight, more preferably of at least 99.5% by weight and most preferably of at least 99.9% by weight is withdrawn as side fraction - preferably from the second middle section - of the second middle dividing-wall distillation column, a C₁₆-fraction having a content of C₁₆-fatty acids of at least 99.0% by weight, more preferably of at least 99.5% by weight and most preferably of at least 99.8% by weight is withdrawn as side fraction of the third distillation column, a C₁₈ₚₗᵤₛ-fraction having a content of C₁₈- and longer fatty acids of at least 99.0% by weight, more preferably of at least 99.5% by weight and most preferably of at least 99.5% by weight is withdrawn as side fraction from the fourth distillation column and a combined C₈₋₁₀-fraction having a content of C₈₋₁₀-fatty acids of at least 98.0% by weight and more preferably of at least 99.0% by weight is withdrawn as side fraction from the first distillation column.

Furthermore, it allows to withdraw from the second middle dividing-wall distillation column of the second particular preferred embodiment, which comprises the use of two middle dividing-wall distillation columns, wherein these middle dividing-wall distillation columns are the second and the third distillation columns, as side fraction - preferably from the top section - a C₁₀-fraction having a content of C₁₀-fatty acids of at least 98.0% by weight, whereas a C₁₂-fraction having a content of C₁₂-fatty acids of at least 98.0% by weight is withdrawn as side fraction - preferably from the second middle section - of the second middle dividing-wall distillation column, a C₁₄-fraction having a content of C₁₄-fatty acids of at least 98.0% by weight is withdrawn as side fraction - preferably from the top section - of the third middle dividing-wall distillation column, a C₁₆-fraction having a content of C₁₆-fatty acids of at least 98.0% is withdrawn as side fraction - preferably from the second section - of the third middle dividing-wall distillation column and a C₁₈ₚₗᵤₛ-fraction having a content of C₁₈- and longer fatty acids of at least 98.0% by weight is withdrawn as side fraction from the fourth distillation column. In addition, a C₈-fraction having a content of C₈-fatty acids of at least 98.0% may be withdrawn as side fraction from the first distillation column and a C₆-fraction having a content of C₆-fatty acids of at least 95.0% by weight may be withdrawn as overhead fraction from the first distillation column. More preferably, during the method of this embodiment a C₁₀-fraction having a content of C₁₀-fatty acids of at least 99.0% by weight, more preferably of at least 99.5% by weight and most preferably of at least 99.8% by weight is withdrawn as side fraction from the second middle dividing-wall distillation column, a C₁₂-fraction having a content of C₁₂-fatty acids of at least 99.0% by weight, more preferably of at least 99.5% by weight and most preferably of at least 99.9% by weight is withdrawn as side fraction from the second middle section of the second middle dividing-wall distillation column, a C₁₄-fraction having a content of C₁₄-fatty acids of at least 99.0% by weight, more preferably of at least 99.5% by weight and most preferably of at least 99.9% by weight is withdrawn as side fraction from the top section of the third middle dividing-wall distillation column, a C₁₆-fraction having a content of C₁₆-fatty acids of at least 99.0% by weight, more preferably of at least 99.5% by weight and most preferably of at least 99.8% by weight is withdrawn as side fraction from the second middle section of the third middle dividing-wall distillation column and a C₁₈ₚₗᵤₛ-fraction having a content of C₁₈- and longer fatty acids of at least 99.0% by weight, more preferably of at least 99.5% by weight and most preferably of at least 99.5% by weight is withdrawn as side fraction from the fourth distillation column. In addition, a C₆-fraction having a content of C₆-fatty acids of at least 95.0% and most preferably of at least 99.8% by weight may be withdrawn as overhead fraction from the first distillation column and a C₈-fraction having a content of C₈-fatty acids of at least 99.0% by weight, more preferably of at least 99.5% by weight and most preferably of at least 99.8% by weight may be withdrawn as side fraction from the first distillation column.

In the third particularly preferred embodiment of the present invention, according to which the method the use of three middle dividing-wall distillation columns, wherein these middle dividing-wall distillation columns are the second, the third and the fifth distillation columns, as side fraction - preferably from the top section - from the second middle dividing-wall distillation column a C₁₀-fraction having a content of C₁₀-fatty acids of at least 98.0% by weight, more preferably of at least 99.0% by weight, even more preferably of at least 99.5% by weight and most preferably of at least 99.8% by weight is withdrawn, wherein a C₁₂-fraction having a content of C₁₂-fatty acids of at least 98.0% by weight, more preferably of at least 99.0% by weight, even more preferably of at least 99.5% by weight and most preferably of at least 99.9% by weight is withdrawn as side fraction - preferably from the second middle section - of the second middle dividing-wall distillation column, whereas from the third middle dividing-wall distillation column a C₁₄-fraction having a content of C₁₄-fatty acids of at least 98.0% by weight, more preferably of at least 99.0% by weight, even more preferably of at least 99.5% by weight and most preferably of at least 99.9% by weight is withdrawn as side fraction - preferably from the top section - and a C₁₆-fraction having a content of C₁₆-fatty acids of at least 98.0% by weight, more preferably of at least 99.0% by weight, even more preferably of at least 99.5% by weight and most preferably of at least 99.8% by weight is withdrawn as side fraction - preferably from the second middle section - of the third middle dividing-wall distillation column. From the fourth distillation column a C₁₈ₚₗᵤₛ-fraction having a content of Cis- and longer fatty acids of at least 98.0% by weight, more preferably of at least 99.0% by weight and most preferably of at least 99.5% by weight is withdrawn as side fraction. Finally, from the fifth middle dividing-wall distillation column a C₆-fraction having a content of C₆-fatty acids of at least 98.0% by weight, more preferably of at least 99.0% by weight, even more preferably of at least 99.5% by weight and most preferably of at least 99.8% by weight is withdrawn as side fraction - preferably from the top section - and a C₈-fraction having a content of C₈-fatty acids of at least 98.0% by weight, more preferably of at least 99.0% by weight, even more preferably of at least 99.5% by weight and most preferably of at least 99.8% by weight is withdrawn as side fraction - preferably from the second middle section - of the fifth middle dividing-wall distillation column.

In order to achieve a particular excellent separation efficiency with a particular low energy consumption, it is suggested in a further development of the idea of the present invention that at least one and preferably all distillation columns comprise at least one element selected from the group consisting of trays, structured packings, random packings and arbitrary combinations of two or more of the aforementioned elements.

Good results are in particular obtained, when at least one and preferably all distillation columns comprise at least one or more structured packings.

Preferably, at least one of the middle dividing-wall distillation columns and more preferably all distillation columns, if more than one is used, each comprise i) two to four, more preferably two to three and most preferably two beds of structured packings in the top section, ii) two to four, more preferably two to three and most preferably two beds of structured packings in each of the middle sections and iii) one to three, more preferably one to two and most preferably one bed of structured packings in the bottom section.

It is further preferred that any of the structured packings is a cross-channel type structured packing, i.e. one which comprises a plurality of adjacent corrugated sheets, which are arranged parallel and in touching contact with each other so that an open space extending from one end to the opposite end of the at least two layers is provided between them so that a fluid may flow through it. The sheets may be metal sheets, which may be perforated by containing a plurality of opening or non-perforated. The sheets may be in particular metal gauze sheets, which may be perforated by containing a plurality of opening or non-perforated. The use of such cross-channel type structured packings, in particular those made of metal gauze sheets, allows to reduce the operating pressure at head of the distillation column and in particular middle dividing-wall distillation column such that the generation of low pressure steam to be withdrawn as purge stream is possible, but with keeping the bottom temperature at below 240°C.

Good results are in particular obtained, when any of the structured packings has a specific area of 60 to 750 m²/m³, more preferably of 120 to 500 m²/m³ and most preferably of 200 to 450 m²/m³.

A further aspect of the present invention is a plant for fractionating a crude composition comprising at least six fatty acids having each a different chain length into at least four fractions, in particular for performing a method of any of the preceding claims, wherein the plant comprises:
i) a first distillation column having an inlet line for the crude composition, an outlet line, which is connected with the head and an outlet line being connected with the bottom of the first distillation column,
ii) a second distillation column having an inlet line being connected with the outlet line being connected with the bottom of the first distillation column, wherein the second middle dividing-wall distillation column has an outlet line being connected with the head and an outlet line being connected with the bottom of the second distillation column,
iii) a third distillation having an inlet line being connected with the outlet line, which is connected with the bottom of the second distillation column, wherein the third middle dividing-wall distillation column has an outlet line being connected with the head and an outlet line being connected with the bottom of the third middle dividing-wall distillation column being located between the head and the bottom of the third distillation column, and
iv) a fourth distillation column having an inlet line being connected with the outlet line, which is connected with the bottom of the third distillation column, wherein the fourth distillation column has an outlet line being connected with the head and an outlet line being connected with the bottom of the fourth distillation column,
wherein at least one of the first distillation column, the second distillation column and the third distillation column is a middle dividing-wall column, which further comprises at least one outlet line being connected with a portion of the middle dividing-wall distillation column being located between the head and the bottom of the second middle dividing-wall distillation column.

In accordance with a variant of a first particular preferred embodiment, in which the plant comprises one middle dividing-wall distillation column, the plant comprises:
i) a first non-dividing-wall distillation column having an inlet line for the crude composition, an outlet line being connected with the head, an outlet line being connected with the bottom of the first non-dividing-wall distillation column and an outlet line being connected with a portion of the first non-dividing-wall distillation column being located between the head and the bottom of the first non-dividing-wall distillation column,
ii) a second middle dividing-wall distillation column having an inlet line being connected with the outlet line, which is connected with the bottom of the first non-dividing-wall distillation column, having an outlet line being connected with the head, an outlet line being connected with the bottom of the second middle dividing-wall distillation column and at least two outlet lines each of which being connected with a portion of the second middle dividing-wall distillation column being located between the head and the bottom of the second middle dividing-wall distillation column,
iii) a third non-dividing-wall distillation having an inlet line being connected with the outlet line, which is connected with the bottom of the second middle dividing-wall distillation column, having an outlet line being connected with the head, an outlet line being connected with the bottom of the third non-dividing-wall distillation column and an outlet line being connected with a portion of the third non-dividing-wall distillation column being located between the head and the bottom of the third non-dividing-wall distillation column, and
iv) a fourth non-dividing-wall distillation column having an inlet line being connected with the outlet line, which is connected with the bottom of the third non-dividing-wall distillation column, having an outlet line being connected with the head, an outlet line being connected with the bottom of the fourth non-dividing-wall distillation column and an outlet line being connected with a portion of the fourth non-dividing-wall distillation column being located between the head and the bottom of the fourth non-dividing-wall distillation column.

In accordance with another variant of the first particular preferred embodiment, in which the plant comprises one middle dividing-wall distillation column, the plant comprises:
i) a first middle dividing-wall distillation column having an inlet line for the crude composition, an outlet line being connected with the head, an outlet line being connected with the bottom of the first middle dividing-wall distillation column and at least one outlet line each of which being connected with a portion of the first middle dividing-wall distillation column being located between the head and the bottom of the first middle dividing-wall distillation column.
ii) a second non-dividing-wall distillation column having an inlet line being connected with the outlet line, which is connected with the bottom of the first middle dividing-wall distillation column, having an outlet line being connected with the head, an outlet line being connected with the bottom of the second non-dividing-wall dividing-wall distillation column and one outlet line being connected with a portion of the second non-dividing-wall distillation column being located between the head and the bottom of the second non-dividing-wall distillation column,
iii) a third non-dividing-wall distillation having an inlet line being connected with the outlet line, which is connected with the bottom of the second non-dividing-wall distillation column, having an outlet line being connected with the head, an outlet line being connected with the bottom of the third non-dividing-wall distillation column and an outlet line being connected with a portion of the third non-dividing-wall distillation column being located between the head and the bottom of the third non-dividing-wall distillation column, and
iv) a fourth non-dividing-wall distillation column having an inlet line being connected with the outlet line, which is connected with the bottom of the third non-dividing-wall distillation column, having an outlet line being connected with the head, an outlet line being connected with the bottom of the fourth non-dividing-wall distillation column and an outlet line being connected with a portion of the fourth non-dividing-wall distillation column being located between the head and the bottom of the fourth non-dividing-wall distillation column.

In accordance with a second particular preferred embodiment, in which the plant comprises two middle dividing-wall distillation columns, the plant comprises:
i) a non-dividing-wall first distillation column having an inlet line for the crude composition, an outlet line being connected with the head, an outlet line being connected with the bottom of the first non-dividing-wall distillation column and an outlet line being connected with a portion of the first non-dividing-wall distillation column being located between the head and the bottom of the first non-dividing-wall distillation column,
ii) a second middle dividing-wall distillation column having an inlet line being connected with the outlet line, which is connected with the bottom of the first non-dividing-wall distillation column, having an outlet line being connected with the head, an outlet line being connected with the bottom of the second middle dividing-wall distillation column and at least two outlet lines each of which being connected with a portion of the second middle dividing-wall distillation column being located between the head and the bottom of the second middle dividing-wall distillation column,
iii) a third middle dividing-wall distillation column having an inlet line being connected with the outlet line, which is connected with the bottom of the second middle dividing-wall distillation column, having an outlet line being connected with the head, an outlet line being connected with the bottom of the third middle dividing-wall distillation column and at least two outlet lines each of which being connected with a portion of the third middle dividing-wall distillation column being located between the head and the bottom of the third middle dividing-wall distillation column, and
iv) a fourth non-dividing-wall distillation column having an inlet line being connected with the outlet line, which is connected with the bottom of the third middle dividing-wall distillation column, having an outlet line being connected with the head, an outlet line being connected with the bottom of the fourth non-dividing-wall distillation column and an outlet line being connected with a portion of the fourth non-dividing-wall distillation column being located between the head and the bottom of the fourth non-dividing-wall distillation column.

In accordance with a third particular preferred embodiment, in which the plant comprises three middle dividing-wall distillation columns, the plant comprises in addition of the four distillation columns of the aforementioned second particular preferred embodiment v) a fifth middle dividing-wall distillation column having an inlet line being connected with the outlet line, which is connected with the head of the first distillation column, wherein the fifth middle dividing-wall distillation column has an outlet line being connected with the head, an outlet line being connected with the bottom and at least two outlet lines being connected with a portion of the middle dividing-wall distillation column being located between the head and the bottom of the fifth middle dividing-wall distillation column. Thus, the plant of this embodiment comprises:
i) a non-dividing-wall first distillation column having an inlet line for the crude composition, an outlet line being connected with the head, an outlet line being connected with the bottom of the first non-dividing-wall distillation column and an outlet line being connected with a portion of the first non-dividing-wall distillation column being located between the head and the bottom of the first non-dividing-wall distillation column,
ii) a second middle dividing-wall distillation column having an inlet line being connected with the outlet line, which is connected with the bottom of the first non-dividing-wall distillation column, having an outlet line being connected with the head, an outlet line being connected with the bottom of the second middle dividing-wall distillation column and at least two outlet lines each of which being connected with a portion of the second middle dividing-wall distillation column being located between the head and the bottom of the second middle dividing-wall distillation column,
iii) a third middle dividing-wall distillation column having an inlet line being connected with the outlet line, which is connected with the bottom of the second middle dividing-wall distillation column, having an outlet line being connected with the head, an outlet line being connected with the bottom of the third middle dividing-wall distillation column and at least two outlet lines each of which being connected with a portion of the third middle dividing-wall distillation column being located between the head and the bottom of the third middle dividing-wall distillation column,
iv) a fourth non-dividing-wall distillation column having an inlet line being connected with the outlet line, which is connected with the bottom of the third middle dividing-wall distillation column, having an outlet line being connected with the head, an outlet line being connected with the bottom of the fourth non-dividing-wall distillation column and an outlet line being connected with a portion of the fourth non-dividing-wall distillation column being located between the head and the bottom of the fourth non-dividing-wall distillation column, and
v) a fifth middle dividing-wall distillation column having an inlet line being connected with the outlet line, which is connected with the head of the first distillation column, wherein the fifth middle dividing-wall distillation column has an outlet line being connected with the head, an outlet line being connected with the bottom and at least two outlet lines being connected with a portion of the middle dividing-wall distillation column being located between the head and the bottom of the fifth middle dividing-wall distillation column.

Preferably, at least one and, if more than one middle dividing-wall distillation column is present, more preferably each of the middle dividing-wall distillation columns comprise a dividing wall extending, seen from the bottom to the top of the middle dividing-wall distillation column, from a point being located at 10 to 40% of the distance from the bottom to the top of the middle dividing-wall distillation column to a point being located at 50 to 90% of the distance from the bottom to the top of the middle dividing-wall distillation column at least essentially vertically downwards so that the middle dividing-wall distillation column comprises above the dividing wall a top section functioning as refinement distillation section, below the dividing wall a bottom section functioning as distillation section, on one side of the dividing wall a first middle section functioning as feed section and on the opposite side of the dividing wall a second middle section functioning as lateral extraction section. Essentially vertically downwards means in accordance with the present invention that the angle between the dividing wall and the length axis of the first dividing-wall distillation column is at most 20°, preferably at most 10°, more preferably at most 5° and most preferably 0°. More preferably, at least one and still more preferably each of the middle dividing-wall distillation columns comprise a dividing wall extending, seen from the bottom to the top of the middle dividing-wall distillation column, from a point being located at 20 to 40% of the distance from the bottom to the top of the middle dividing-wall distillation column to a point being located at 50 to 80% of the distance from the bottom to the top of the middle dividing-wall distillation column at least essentially vertically downwards. Most preferably, at least one and still more preferably each of the middle dividing-wall distillation columns comprise a dividing wall extending, seen from the bottom to the top of the middle dividing-wall distillation column, from a point being located at 25 to 35% of the distance from the bottom to the top of the middle dividing-wall distillation column to a point being located at 55 to 75% of the distance from the bottom to the top of the middle dividing-wall distillation column at least essentially vertically downwards.

Good results in terms of high separation efficiency at low energy consumption are in particular obtained, when in at least one and preferably each of the middle dividing-wall distillation columns the dividing wall extends over 20 to 80%, more preferably over 30 to 70% and most preferably over 30 to 60% of the height of the middle dividing-wall distillation column. The height of the middle dividing-wall distillation column is defined in this connection as the straight distance between the top and the bottom of the respective middle dividing-wall distillation column.

In a further development of the idea of the present invention, the inlet line of at least one and preferably each of the middle dividing-wall distillation columns is connected with the first middle section of the respective middle dividing-wall distillation column.

Preferably, in at least one and preferably each of the middle dividing-wall distillation columns one of the at least two outlet lines each of which being connected with a portion of the middle dividing-wall distillation column being located between the head and the bottom of the second middle dividing-wall distillation column is connected with the top section and another one of these at least two outlet lines is connected with the second middle section of the respective middle dividing-wall distillation column.

It is further preferred that at least one and preferably each of the middle dividing-wall distillation columns comprise a purge withdrawal line in the top section between the outlet line being connected with the top section and the outlet line being connected with the middle section of the respective middle dividing-wall distillation column.

In accordance with a further particular preferred embodiment of the present invention, at least one and preferably all distillation columns of the plant comprise at least one element selected from the group consisting of trays, structured packings, random packings and arbitrary combinations of two or more of the aforementioned elements.

Preferably, at least one and more preferably all distillation columns comprise at least one or more structured packings.

Good results are in particular obtained, when one and, if more than one middle dividing-wall distillation column is present, all middle dividing-wall distillation columns each comprise i) two to four, preferably two to three and more preferably two beds of structured packings in the top section, ii) two to four, preferably two to three and more preferably two beds of structured packings in each of the middle sections and iii) one to three, preferably one to two and more preferably one bed of structured packings in the bottom section.

In a further development of the idea of the present invention, it is suggested that the structured packing is a cross-channel type structured packing which comprises a plurality of adjacent corrugated sheets, which are arranged parallel and in touching contact with each other so that an open space extending from one end to the opposite end of the at least two layers is provided between them so that a fluid may flow through it. The sheets may be metal sheets, which may be perforated by containing a plurality of opening or non-perforated. The sheets may be in particular metal gauze sheets, which may be perforated by containing a plurality of opening or non-perforated.

Good results are in particular obtained, when any of the structured packings has a specific area of 60 to 750 m²/m³, more preferably of 120 to 500 m²/m³ and most preferably of 200 to 450 m²/m³.

Subsequently, the present invention is illustrated by means of exemplary, but nonlimiting figures, in which:
- Fig. 1: is a schematic view of a plant for fractionating palm kernel fatty acid in accordance with the prior art.
- Fig. 2: is a schematic view of a plant for fractionating palm kernel fatty acid in accordance with one embodiment of the present invention.
- Fig. 3: is a schematic view of a plant for fractionating palm kernel fatty acid in accordance with another embodiment of the present invention.
- Fig. 4: is a schematic view of a plant for fractionating palm kernel fatty acid in accordance with another embodiment of the present invention.

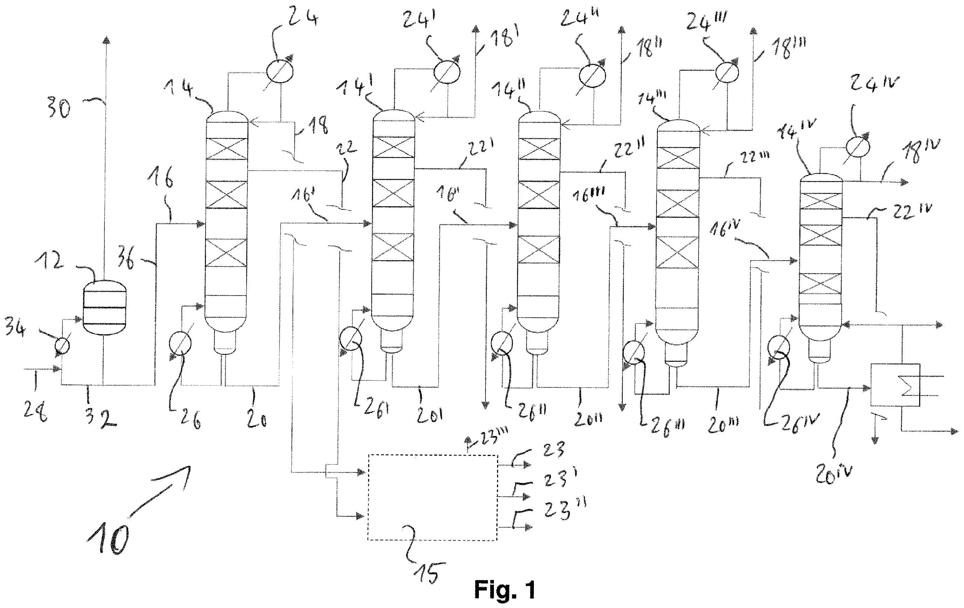

Fig. 1 shows schematically a plant 1 for fractionating palm kernel fatty acid in accordance with the prior art. The plant 1 comprises, from its upstream to its downstream end, a drier or deaerator 12, respectively, five distillation column 14 to 14^{iv} and a multipurpose fractionation unit 15 comprising two further distillation columns (not shown). Each of the five distillation columns 14 to 14^{iv} comprises an inlet line 16 to 16^{iv}, an outlet line 18 to 18^{iv} being connected with the head of the respective distillation column 14 to 14^{iv}, an outlet line 20 to 20^{iv} being connected with the bottom of the respective distillation column 14 to 14^{iv} and an outlet 22 to 22^{iv} being connected with a portion of the respective distillation column 14 to 14^{iv} being located between its head and its bottom. Each of the inlet lines 16' to 16^{iv} of the second to fifth distillation column 14' to 14^{iv} is connected with the outlet line 20 to 20ⁱⁱⁱ being connected with the bottom of the adjacent upstream distillation column 14 to 14ⁱⁱⁱ. The outlet lines 18 and 22 of the first distillation column 14 are connected with the multipurpose fractionation unit 15, which has four outlet lines 23, 23', 23", 23"'. Each of the distillation columns comprises a condenser 24 to 24^{iv} being in fluid communication with the head and a reboiler 26 to 26^{iv} being in fluid communication with the bottom of the non-dividing-wall distillation column 14 to 14^{iv}. The drier or deaerator 12, respectively, is connected with a feed inlet line 28, a gas outlet line 30, a recirculation line 32 comprising a reboiler 34 and an outlet line 36, which is connected with the inlet line 16 to the first distillation column 14.

During the operation of this plant 1, crude palm kernel fatty acid is continuously fed via the feed inlet line 28 and the recirculation line 32 into the drier or deaerator 12, respectively. Gas separated within the drier or deaerator 12, respectively, is withdrawn from the drier or deaerator 12, respectively, via the gas outlet line 30, whereas the dried and deaerated crude palm kernel fatty acid is fed via the inlet line 16 into the first distillation column 14. The crude palm kernel fatty acid is distilled in the first distillation column 14 so as to obtain an overhead fraction as C₆-fraction of the palm kernel fatty acid, which is withdrawn from the first distillation column 14 via the outlet line 18, a side fraction as C₈₋₁₀-fraction of the palm kernel fatty acid, which is withdrawn from the first distillation column 14 via the outlet line 22, and a bottom fraction with the remaining fatty acids, which is withdrawn from the first distillation column 14 via the outlet line 20. The C₆-fraction and the C₈₋₁₀-fraction of the palm kernel fatty acid are fed via lines 18 and 22 into the multipurpose fractionation unit 15, in which these fractions are fractionated into a C₆-fraction, a C₈-fraction and a C₁₀-fraction, which are withdrawn via the outlet lines 23, 23', 23". Moreover, a purge stream is withdrawn from the multipurpose fractionation unit 15 via the outlet line 23"'. The bottom fraction of the first distillation column 14 is fed via lines 20, 16' into the second distillation column 14', in which it is distilled so as to obtain a light end as overhead fraction, which is withdrawn from the second distillation column 14' via the outlet line 18', a side fraction as C₁₂-fraction of the palm kernel fatty acid, which is withdrawn from the second distillation column 14' via the outlet line 22', and a bottom fraction with the remaining fatty acids, which is withdrawn from the second distillation column 14' via the outlet line 20'. The bottom fraction of the second distillation column 14' is fed via lines 20', 16" into the third distillation column 14", in which it is distilled so as to obtain a light end as overhead fraction, which is withdrawn from the third distillation column 14" via the outlet line 18", a side fraction as C₁₄-fraction of the palm kernel fatty acid, which is withdrawn from the third distillation column 14" via the outlet line 22", and a bottom fraction with the remaining fatty acids, which is withdrawn from the third distillation column 14" via the outlet line 20". Likewise, the bottom fraction of the third distillation column 14" is fed via lines 20", 16'" into the fourth distillation column 14"', in which it is distilled so as to obtain a light end as overhead fraction, which is withdrawn from the fourth distillation column 14'" via the outlet line 18"', a side fraction as C₁₆-fraction of the palm kernel fatty acid, which is withdrawn from the fourth distillation column 14"' via the outlet line 22"', and a bottom fraction with the remaining fatty acids, which is withdrawn from the fourth distillation column 14'" via the outlet line 20"'. Finally, the bottom fraction of the fourth distillation column 14'" is fed via lines 20"', 16^{iv} into the fifth distillation column 14^{iv}, in which it is distilled so as to obtain a light end as overhead fraction, which is withdrawn from the fourth distillation column 14^{iv} via the outlet line 18^{iv}, a side fraction as C₁₈ₚₗᵤₛ-fraction of the palm kernel fatty acid, which is withdrawn from the fifth distillation column 14^{iv} via the outlet line 22^{iv}, and a bottom fraction with the residuals.

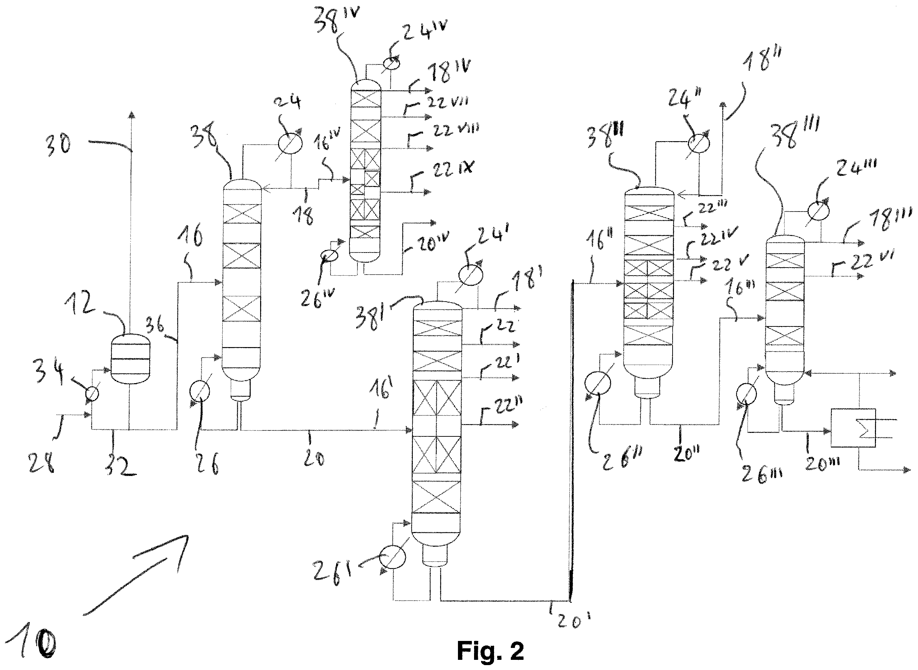

Fig. 2 shows schematically a plant 10 for fractionating palm kernel fatty acid in accordance with one embodiment of the present invention, which corresponds to the third particular preferred embodiment as described above. The plant 10 comprises a drier or deaerator 12, respectively, a first distillation column 38 embodied as non-dividing-wall column, a second distillation column 38' embodied as middle dividing-wall column, a third distillation column 38" embodied as middle dividing-wall column, a fourth distillation column 38'" embodied as non-dividing-wall column as well as a fifth distillation column 38^{iv} embodied as middle dividing-wall column. The first distillation column 38 has an inlet line 16 for the crude composition, an outlet line 18 being connected with the head and an outlet line 20 being connected with the bottom of the first distillation column 38. The inlet line 16 of the first distillation column 38 is connected with the outlet line 36 of the drier or deaerator 12, respectively, which is embodied as in figure 1, i.e. which is connected with a feed inlet line 28, a gas outlet line 30, a recirculation line 32 comprising a reboiler 34 and the outlet line 36. The second middle dividing-wall distillation column 38' has an inlet line 16' being connected with the outlet line 20, which is connected with the bottom of the first distillation column 38, wherein the second middle dividing-wall distillation column 38' has an outlet line 18' being connected with the head, an outlet line 20' being connected with the bottom and three outlet lines 22, 22', 22" each of which being connected with a portion of the middle dividing-wall distillation column being located between the head and the bottom of the second middle dividing-wall distillation column 38'. Likewise, the third middle dividing-wall distillation 38" has an inlet line 16" being connected with the outlet line 20', which is connected with the bottom of the second middle dividing-wall distillation column 38', wherein the third middle dividing-wall distillation 38" has an outlet line 18" being connected with the head, an outlet line 20" being connected with the bottom and three outlet lines 22"', 22^{iv}, 22^{v} each of which being connected with a portion of the third middle dividing-wall distillation column 38" being located between the head and the bottom of the third middle dividing-wall distillation column 38". The fourth distillation column 38'" has an inlet line 16'" being connected with the outlet line 20", which is connected with the bottom of the third middle dividing-wall distillation column 38", wherein the fourth distillation column 38'" has an outlet line 18'" being connected with the head, an outlet line 20'" being connected with the bottom and an outlet line 22^{vi} being connected with a portion of the fourth distillation column 38'" being located between the head and the bottom of the fourth distillation column 38"'. Finally, the fifth middle dividing-wall distillation 38^{iv} has an inlet line 16^{iv} being connected with the outlet line 18, which is connected with the head of the first dividing-wall distillation column 38, wherein the fifth middle dividing-wall distillation 38^{iv} has an outlet line 18^{iv} being connected with the head, an outlet line 20^{iv} being connected with the bottom and three outlet lines 22^{vii}, 22^{vii}, 22^{viii} each of which being connected with a portion of the fifth middle dividing-wall distillation column 38^{iv} being located between the head and the bottom of the fifth middle dividing-wall distillation column 38^{iv}.

During the operation of this plant 10, crude palm kernel fatty acid is continuously fed via the feed inlet line 28 and the recirculation line 32 into the drier or deaerator 12, respectively. Gas separated within the drier or deaerator 12, respectively, is withdrawn from the drier or deaerator 12, respectively, via the gas outlet line 30, whereas the dried and deaerated crude palm kernel fatty acid is fed via the inlet line 16 into the first distillation column 38. The crude palm kernel fatty acid is distilled in the first distillation column 38 so as to obtain an overhead fraction including C₆₋₈-fatty acids, which is withdrawn from the first distillation column 38 via the outlet line 18, and a bottom fraction with the remaining fatty acids, which is withdrawn from the first distillation column 38 via the outlet line 20. The bottom fraction of the first distillation column 38 is fed via lines 20, 16' into the second middle dividing-wall distillation column 38', in which it is distilled so as to obtain a light end as overhead fraction, which is withdrawn from the second middle dividing-wall distillation column 38' via the outlet line 18', a side fraction as C₁₀-fraction of the palm kernel fatty acid, which is withdrawn from the second middle dividing-wall distillation column 38' via the outlet line 22, a purge stream, which is withdrawn from the second middle dividing-wall distillation column 38' via the outlet line 22', a side fraction as C₁₂-fraction of the palm kernel fatty acid, which is withdrawn from the second middle dividing-wall distillation column 38' via the outlet line 22", and a bottom fraction with the remaining fatty acids, which is withdrawn from the second middle dividing-wall distillation column 38' via the outlet line 20'. The bottom fraction of the second distillation column 38' is fed via lines 20', 16" into the third middle dividing-wall distillation column 38", in which it is distilled so as to obtain a light end as overhead fraction, which is withdrawn from the third middle dividing-wall distillation column 38" via the outlet line 18", a side fraction as C₁₄-fraction of the palm kernel fatty acid, which is withdrawn from the third middle dividing-wall distillation column 38" via the outlet line 22"', a purge stream, which is withdrawn from the third middle dividing-wall distillation column 38" via the outlet line 22^{iv}, a side fraction as C₁₆-fraction of the palm kernel fatty acid, which is withdrawn from the third middle dividing-wall distillation column 38" via the outlet line 22^{v}, and a bottom fraction with the remaining fatty acids, which is withdrawn from the third middle dividing-wall distillation column 38" via the outlet line 20". The bottom fraction of the third distillation column 38" is fed via lines 20", 16'" into the fourth distillation column 38"', in which it is distilled so as to obtain a light end as overhead fraction, which is withdrawn from the fourth distillation column 38"' via the outlet line 18"', a side fraction as C₁₈ₚₗᵤₛ-fraction of the palm kernel fatty acid, which is withdrawn from the fourth distillation column 38'" via the outlet line 22^{vi}, and a bottom fraction, which is withdrawn from the fourth distillation column 38'" via the outlet line 20"'. Finally, the head fraction of the first distillation column 38 is fed via lines 18, 16^{iv} into the fifth middle dividing-wall distillation column 38^{iv}, in which it is distilled so as to obtain a light end as overhead fraction, which is withdrawn from the fifth middle dividing-wall distillation column 38^{iv} via the outlet line 18^{iv}, a side fraction as C₆-fraction of the palm kernel fatty acid, which is withdrawn from the fifth middle dividing-wall distillation column 38^{iv} via the outlet line 22^{vii}, a purge stream, which is withdrawn from the fifth middle dividing-wall distillation column 38^{iv} via the outlet line 22^{viii}, a side fraction as C₈-fraction of the palm kernel fatty acid, which is withdrawn from the fifth middle dividing-wall distillation column 38^{iv} via the outlet line 22^{vix}, and a purge as bottom fraction, which is withdrawn from the fifth middle dividing-wall distillation column 38^{iv} via the outlet line 20^{iv}.

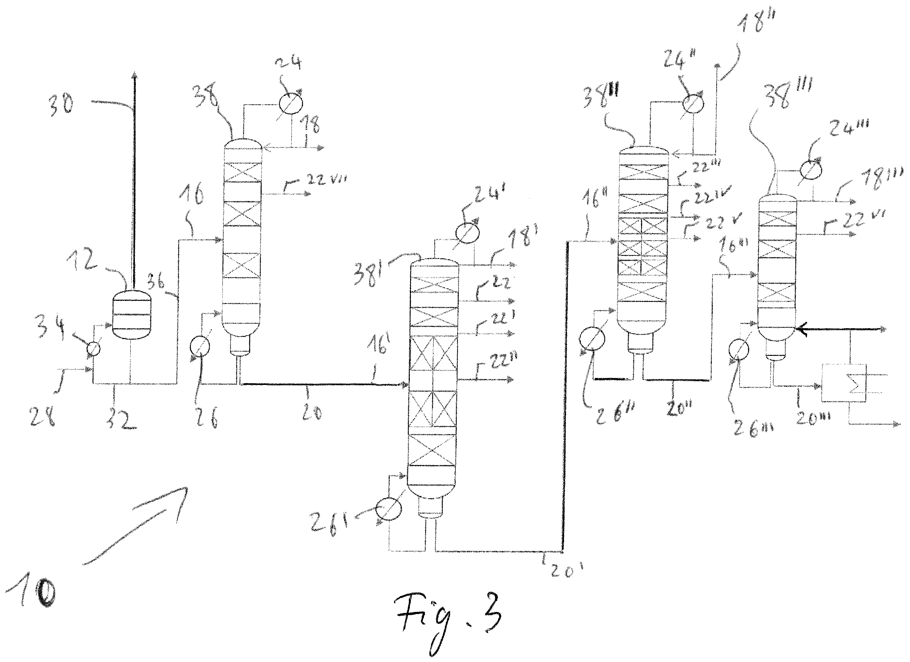

Fig. 3 shows schematically a plant 10 for fractionating palm kernel fatty acid in accordance with another embodiment of the present invention, which corresponds to the second particular preferred embodiment as described above. The plant 10 corresponds to that of figure 2 except that the fifth distillation column 38^{iv} is absent. Instead, the first distillation column 18 comprises in addition to the outlet line 18 of the head and the outlet line 20 of the bottom an outlet line 22^{vii} of the side of the first distillation column 18.

The operation performed during the operation of this plant 10 is the same as that performed during the operation of the plant 10 shown in figure 2 except that the head product of the first distillation column 18 withdrawn via line 18 is a C₆-fraction of the palm kernel fatty acid having a purity of about 95% and furthermore from the side of the first distillation column 18 via line 22^{vii} a C₈-fraction of the palm kernel fatty acid having a purity of about 99.5% is withdrawn.

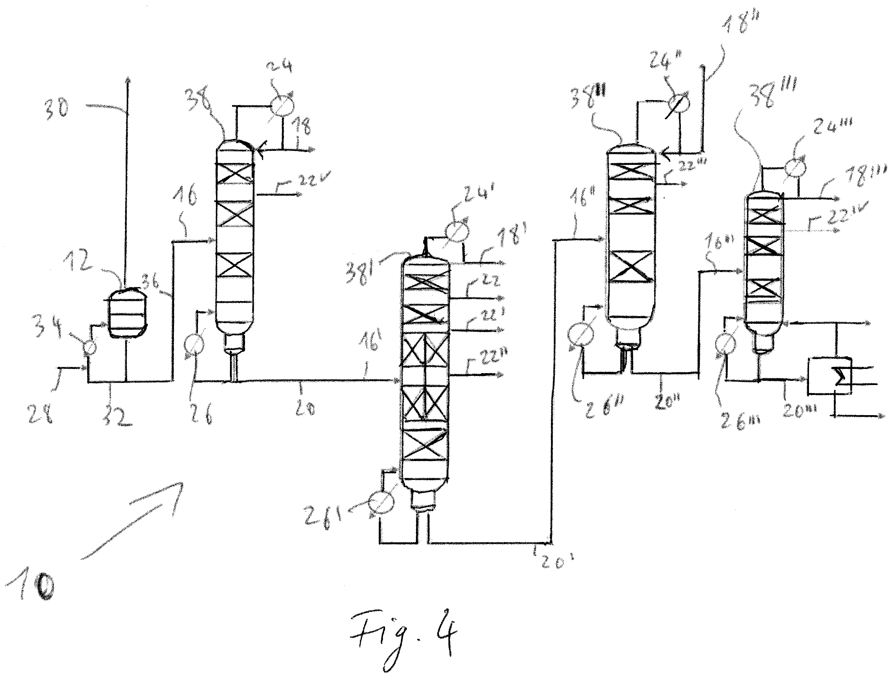

Fig. 4 shows schematically a plant 10 for fractionating palm kernel fatty acid in accordance with another embodiment of the present invention, which corresponds to the first particular preferred embodiment as described above. The plant 10 corresponds to that of figure 3 except that the third distillation column 38" is not a middle dividing-wall column, but a non-dividing-wall column.

During the operation of this plant 10, crude palm kernel fatty acid is continuously fed via the feed inlet line 28 and the recirculation line 32 into the drier or deaerator 12, respectively. Gas separated within the drier or deaerator 12, respectively, is withdrawn from the drier or deaerator 12, respectively, via the gas outlet line 30, whereas the dried and deaerated crude palm kernel fatty acid is fed via the inlet line 16 into the first distillation column 38. The crude palm kernel fatty acid is distilled in the first distillation column 38 so as to obtain an overhead fraction includ-inglights, which is withdrawn from the first distillation column 38 via the outlet line 18, a C₈₋₁₀-fatty acids fraction withdrawn via the outlet line 22^{v} and a bottom fraction with the remaining fatty acids, which is withdrawn from the first distillation column 38 via the outlet line 20. The bottom fraction of the first distillation column 38 is fed via lines 20, 16' into the second middle dividing-wall distillation column 38', in which it is distilled so as to obtain a light end as overhead fraction, which is withdrawn from the second middle dividing-wall distillation column 38' via the outlet line 18', a side fraction as C₁₂-fraction of the palm kernel fatty acid, which is withdrawn from the second middle dividing-wall distillation column 38' via the outlet line 22, a purge stream, which is withdrawn from the second middle dividing-wall distillation column 38' via the outlet line 22', a side fraction as C₁₄-fraction of the palm kernel fatty acid, which is withdrawn from the second middle dividing-wall distillation column 38' via the outlet line 22", and a bottom fraction with the remaining fatty acids, which is withdrawn from the second middle dividing-wall distillation column 38' via the outlet line 20'. The bottom fraction of the second distillation column 38' is fed via lines 20', 16" into the third non-dividing-wall distillation column 38", in which it is distilled so as to obtain a light end as overhead fraction, which is withdrawn from the third non-dividing-wall distillation column 38" via the outlet line 18", a side fraction as C₁₆-fraction of the palm kernel fatty acid, which is withdrawn from the third non-dividing-wall distillation column 38" via the outlet line 22'" and a bottom fraction with the remaining fatty acids, which is withdrawn from the third non-dividing-wall distillation column 38" via the outlet line 20". The bottom fraction of the third distillation column 38" is fed via lines 20", 16'" into the fourth distillation column 38"', in which it is distilled so as to obtain a light end as overhead fraction, which is withdrawn from the fourth distillation column 38'" via the outlet line 18"', a side fraction as C₁₈ₚₗᵤₛ-fraction of the palm kernel fatty acid, which is withdrawn from the fourth distillation column 38'" via the outlet line 22^{iv}, and a bottom fraction which is withdrawn from the fourth distillation column 38'" via the outlet line 20"'.

### Reference Numeral List

- 1: Plant according to the prior art
- 10: Plant according to the present invention
- 12: Drier or deaerator, respectively
- 14 to 14^{iv}: Distillation column of the prior art plant
- 15: Multipurpose fractionation unit of the prior art plant
- 16 to 16^{iv}: Inlet line to distillation column
- 18 to 18^{iv}: Outlet line of head of distillation column
- 20 to 20^{iv}: Outlet line of bottom of distillation column
- 22 to 22^{ix}: Outlet line of side of distillation column
- 23, 23', 23", 23'": Outlet line of multipurpose fractionation unit
- 24 to 24iv: Condenser
- 26 to 26iv: Reboiler
- 28: Feed inlet line
- 30: Outlet line of drier
- 32: Recirculation line of drier
- 34: Reboiler of drier
- 36: Outlet line of drier
- 38 to 38^{iv}: Distillation column of the present invention

## Claims

1. A method for fractionating a crude composition comprising at least six fatty acids having each a different chain length into at least four fractions, wherein the method comprises the following steps:
i) feeding the crude composition into a first distillation column and distilling it so as to obtain at least an overhead fraction and a bottom fraction,
ii) feeding the bottom fraction of the first distillation column to a second distillation column and distilling it so as to obtain at least an overhead fraction and a bottom fraction,
iii) feeding the bottom fraction of the second distillation column to a third distillation column and distilling it so as to obtain at least an overhead fraction and a bottom fraction and
iv) feeding the bottom fraction of the third distillation column to a fourth distillation column and distilling it so as to obtain at least an overhead fraction, a side fraction and a bottom fraction,
wherein at least one of the first distillation column, the second distillation column and the third distillation column is a middle dividing-wall column, from which also at least one side fraction is obtained.

2. The method of claim 1, which comprises the following steps:
i) feeding the crude composition into a first non-dividing-wall distillation column and distilling it so as to obtain at least an overhead fraction, a side fraction and a bottom fraction,
ii) feeding the bottom fraction of the first non-dividing-wall distillation column to a second middle dividing-wall distillation column and distilling it so as to obtain at least an overhead fraction, at least two side fractions and a bottom fraction,
iii) feeding the bottom fraction of the second middle dividing-wall distillation column to a third non-dividing-wall distillation column and distilling it so as to obtain at least an overhead fraction, a side fraction and a bottom fraction and
iv) feeding the bottom fraction of the third non-dividing-wall distillation column to a fourth non-dividing-wall distillation column and distilling it so as to obtain at least an overhead fraction, a side fraction and a bottom fraction.

3. The method of claim 1, which comprises the following steps:
i) feeding the crude composition into a first middle dividing-wall distillation column and distilling it so as to obtain at least an overhead fraction, at least two side fractions and a bottom fraction,
ii) feeding the bottom fraction of the first middle dividing-wall distillation column to a second non-dividing-wall distillation column and distilling it so as to obtain at least an overhead fraction, a side fraction and a bottom fraction,
iii) feeding the bottom fraction of the second non-dividing-wall distillation column to a third non-dividing-wall distillation column and distilling it so as to obtain at least an overhead fraction, a side fraction and a bottom fraction and
iv) feeding the bottom fraction of the third non-dividing-wall distillation column to a fourth non-dividing-wall distillation column and distilling it so as to obtain at least an overhead fraction, a side fraction and a bottom fraction.

4. The method of claim 1, which comprises the following steps:
i) feeding the crude composition into a first non-dividing-wall distillation column and distilling it so as to obtain at least an overhead fraction, a side fraction and a bottom fraction,
ii) feeding the bottom fraction of the first non-dividing-wall distillation column to a second middle dividing-wall distillation column and distilling it so as to obtain at least an overhead fraction, at least two side fractions and a bottom fraction,
iii) feeding the bottom fraction of the second middle dividing-wall distillation column to a third middle dividing-wall distillation column and distilling it so as to obtain at least an overhead fraction, at least two side fractions and a bottom fraction and
iv) feeding the bottom fraction of the third middle dividing-wall distillation column to a fourth non-dividing-wall distillation column and distilling it so as to obtain at least an overhead fraction, a side fraction and a bottom fraction.

5. The method of claim 4, further comprising the step v) of feeding the overhead fraction of the first non-dividing-wall distillation column to a fifth middle dividing-wall distillation column and distilling it so as to obtain an overhead fraction, at least two side fractions and a bottom fraction.

6. The method of any of the preceding claims, wherein at least one and preferably each of the middle dividing-wall distillation columns comprise a dividing wall extending, seen from the bottom to the top of the middle dividing-wall distillation column, from a point being located at 10 to 40% of the distance from the bottom to the top of the middle dividing-wall distillation column to a point being located at 50 to 90% of the distance from the bottom to the top of the middle dividing-wall distillation column at least essentially vertically downwards so that the middle dividing-wall distillation column comprises above the dividing wall a top section functioning as refinement distillation section, below the dividing wall a bottom section functioning as distillation section, on one side of the dividing wall a first middle section functioning as feed section and on the opposite side of the dividing wall a second middle section functioning as lateral extraction section, wherein essentially vertically downwards means that the angle between the dividing wall and the length axis of the middle dividing-wall distillation column is at most 20°, preferably at most 10°, more preferably at most 5° and most preferably 0°, and, wherein in at least one and preferably each of the middle dividing-wall distillation columns the dividing wall extends over 20 to 80%, preferably over 30 to 70% and more preferably over 30 to 60% of the height of the middle dividing-wall distillation column, wherein the height of the middle dividing-wall distillation column is the straight distance between the top and the bottom of the middle dividing-wall distillation column.

7. The method of claim 6, wherein the feed of at least one and preferably each of the middle dividing-wall distillation columns is fed into the first middle section of the middle dividing-wall distillation column, wherein from at least one and preferably each of the middle dividing-wall distillation columns one of the at least two side fractions is withdrawn from the top section and another one of the at least two side fractions is withdrawn from the second middle section of the middle dividing-wall distillation column, and, wherein from at least one and preferably each of the middle dividing-wall distillation columns a purge is withdrawn from the top section of the middle dividing-wall distillation column between the side fraction being withdrawn from the top section and the side fraction being withdrawn from the second middle section of the middle dividing-wall distillation column.

8. The method of any of the preceding claims, wherein the crude composition contains palm kernel fatty acid and/or coconut fatty acid and preferably consists of palm kernel fatty acid and/or coconut fatty acid.

9. The method of claim 4 and claim 8 and any of claims 6 to 7, wherein from the second middle dividing-wall distillation column a C₁₀-fraction having a content of C₁₀-fatty acids of at least 98.0% by weight, preferably of at least 99.0% by weight and more preferably of at least 99.5% by weight is withdrawn as side fraction preferably from the top section and a C₁₂-fraction having a content of C₁₂-fatty acids of at least 98.0% by weight, preferably of at least 99.0% by weight and more preferably of at least 99.5% by weight is withdrawn as side fraction preferably from the second middle section of the second middle dividing-wall distillation column, wherein from the third middle dividing-wall distillation column a C₁₄-fraction having a content of C₁₄-fatty acids of at least 98.0% by weight, preferably of at least 99.0% by weight and more preferably of at least 99.5% by weight is withdrawn as side fraction preferably from the top section and a C₁₆-fraction having a content of C₁₆-fatty acids of at least 98.0% by weight, preferably of at least 99.0% by weight and more preferably of at least 99.5% by weight is withdrawn as side fraction preferably from the second middle section of the third middle dividing-wall distillation column, and wherein from the fourth distillation column a C₁₈ₚₗᵤₛ-fraction having a content of C₁₈- and longer fatty acids of at least 98.0% by weight and preferably of at least 99.0% by weight is withdrawn as side fraction.

10. The method of any of the preceding claims, wherein at least one and preferably all distillation columns middle dividing-wall distillation columns each comprise two to four, preferably two to three and more preferably two beds of structured packings in the top section, two to four, preferably two to three and more preferably two beds of structured packings in each of the middle sections and one to three, preferably one to two and more preferably one bed of structured packings in the bottom section.

11. A plant for fractionating a crude composition comprising at least six fatty acids having each a different chain length into at least four fractions, in particular for performing a method of any of the preceding claims, wherein the plant comprises:
i) a first distillation column having an inlet line for the crude composition, an outlet line being connected with the head and an outlet line being connected with the bottom of the first distillation column,
ii) a second distillation column having an inlet line being connected with the outlet line, which is connected with the bottom of the first distillation column, having an outlet line being connected with the head and an outlet line being connected with the bottom of the second distillation column,
iii) a third distillation having an inlet line being connected with the outlet line, which is connected with the bottom of the second distillation column, having an outlet line being connected with the head and an outlet line being connected with the bottom of the third distillation column, and
iv) a fourth distillation column having an inlet line being connected with the outlet line, which is connected with the bottom of the third distillation column, having an outlet line being connected with the head and an outlet line being connected with the bottom of the fourth distillation column,
wherein at least one of the first distillation column, the second distillation column and the third distillation column is a middle dividing-wall column, which further comprises at least one outlet line being connected with a portion of the middle dividing-wall distillation column being located between the head and the bottom of the second middle dividing-wall distillation column.

12. The plant of claim 11, which comprises:
i) a first non-dividing-wall distillation column having an inlet line for the crude composition, an outlet line being connected with the head, an outlet line being connected with the bottom of the first non-dividing-wall distillation column and an outlet line being connected with a portion of the first non-dividing-wall distillation column being located between the head and the bottom of the first non-dividing-wall distillation column,
ii) a second middle dividing-wall distillation column having an inlet line being connected with the outlet line, which is connected with the bottom of the first non-dividing-wall distillation column, having an outlet line being connected with the head, an outlet line being connected with the bottom of the second middle dividing-wall distillation column and at least two outlet lines each of which being connected with a portion of the second middle dividing-wall distillation column being located between the head and the bottom of the second middle dividing-wall distillation column,
iii) a third non-dividing-wall distillation having an inlet line being connected with the outlet line, which is connected with the bottom of the second middle dividing-wall distillation column, having an outlet line being connected with the head, an outlet line being connected with the bottom of the third non-dividing-wall distillation column and an outlet line being connected with a portion of the third non-dividing-wall distillation column being located between the head and the bottom of the third non-dividing-wall distillation column, and
iv) a fourth non-dividing-wall distillation column having an inlet line being connected with the outlet line, which is connected with the bottom of the third non-dividing-wall distillation column, having an outlet line being connected with the head, an outlet line being connected with the bottom of the fourth non-dividing-wall distillation column and an outlet line being connected with a portion of the fourth non-dividing-wall distillation column being located between the head and the bottom of the fourth non-dividing-wall distillation column.

13. The plant of claim 11, which comprises:
i) a first middle dividing-wall distillation column having an inlet line for the crude composition, an outlet line being connected with the head, an outlet line being connected with the bottom of the first middle dividing-wall distillation column and at least one outlet line each of which being connected with a portion of the first middle dividing-wall distillation column being located between the head and the bottom of the first middle dividing-wall distillation column.
ii) a second non-dividing-wall distillation column having an inlet line being connected with the outlet line, which is connected with the bottom of the first middle dividing-wall distillation column, having an outlet line being connected with the head, an outlet line being connected with the bottom of the second non-dividing-wall dividing-wall distillation column and one outlet line being connected with a portion of the second non-dividing-wall distillation column being located between the head and the bottom of the second non-dividing-wall distillation column,
iii) a third non-dividing-wall distillation having an inlet line being connected with the outlet line, which is connected with the bottom of the second non-dividing-wall distillation column, having an outlet line being connected with the head, an outlet line being connected with the bottom of the third non-dividing-wall distillation column and an outlet line being connected with a portion of the third non-dividing-wall distillation column being located between the head and the bottom of the third non-dividing-wall distillation column, and
iv) a fourth non-dividing-wall distillation column having an inlet line being connected with the outlet line, which is connected with the bottom of the third non-dividing-wall distillation column, having an outlet line being connected with the head, an outlet line being connected with the bottom of the fourth non-dividing-wall distillation column and an outlet line being connected with a portion of the fourth non-dividing-wall distillation column being located between the head and the bottom of the fourth non-dividing-wall distillation column.

14. The plant of claim 11, which comprises:
i) a non-dividing-wall first distillation column having an inlet line for the crude composition, an outlet line being connected with the head, an outlet line being connected with the bottom of the first non-dividing-wall distillation column and an outlet line being connected with a portion of the first non-dividing-wall distillation column being located between the head and the bottom of the first non-dividing-wall distillation column,
ii) a second middle dividing-wall distillation column having an inlet line being connected with the outlet line, which is connected with the bottom of the first non-dividing-wall distillation column, having an outlet line being connected with the head, an outlet line being connected with the bottom of the second middle dividing-wall distillation column and at least two outlet lines each of which being connected with a portion of the second middle dividing-wall distillation column being located between the head and the bottom of the second middle dividing-wall distillation column,
iii) a third middle dividing-wall distillation column having an inlet line being connected with the outlet line, which is connected with the bottom of the second middle dividing-wall distillation column, having an outlet line being connected with the head, an outlet line being connected with the bottom of the third middle dividing-wall distillation column and at least two outlet lines each of which being connected with a portion of the third middle dividing-wall distillation column being located between the head and the bottom of the third middle dividing-wall distillation column, and
iv) a fourth non-dividing-wall distillation column having an inlet line being connected with the outlet line, which is connected with the bottom of the third middle dividing-wall distillation column, having an outlet line being connected with the head, an outlet line being connected with the bottom of the fourth non-dividing-wall distillation column and an outlet line being connected with a portion of the fourth non-dividing-wall distillation column being located between the head and the bottom of the fourth non-dividing-wall distillation column.

15. The plant of claim 14, further comprising a fifth middle dividing-wall distillation column having an inlet line being connected with the outlet line, which is connected with the head of the first distillation column, wherein the fifth middle dividing-wall distillation column has an outlet line being connected with the head, an outlet line being connected with the bottom and at least two outlet lines being connected with a portion of the fifth middle dividing-wall distillation column being located between the head and the bottom of the fifth middle dividing-wall distillation column.
